# EUROPÄISCHE PATENTSCHRIFT

(11) **EP 0 731 692 B1**
(45) Veröffentlichungstag und Bekanntmachung des Hinweises auf die Patenterteilung: **09.08.2000**
(21) Anmeldenummer: 95902778.0
(22) Anmeldetag: 23.11.1994
(51) Int. Cl.: A61K 9/20

(54) **VORRICHTUNG ZUR KONTROLLIERTEN FREISETZUNG VON WIRKSTOFFEN**
DEVICE FOR EFFECTING THE CONTROLLED RELEASE OF ACTIVE SUBSTANCES
PROCEDE DE LIBERATION CONTROLEE DE PRINCIPES ACTIFS

(30) Priorität: 04.12.1993 DE 4341442
(43) Veröffentlichungstag der Anmeldung: 18.09.1996
(73) Patentinhaber: LTS Lohmann Therapie-Systeme AG, 56626 Andernach (DE)
(72) Erfinder: CREMER, Karsten, D-53119 Bonn (DE)
(74) Vertreter: Flaccus, Rolf-Dieter, Dr.
(86) Internationale Anmeldenummer: EP9403865
(87) Internationale Veröffentlichungsnummer: WO9515156

(56) Entgegenhaltungen:
- WO-A-94/07470
- GB-A- 994 742

## Beschreibung

Die Erfindung betrifft eine Vorrichtung zur kontrollierten Freisetzung von Wirkstoffen in flüssigen Medien aus einer wirkstoffhaltigen, Wirkstoffabgabeflächen aufweisenden Matrix und einer diese mindestens teilweise abdeckenden erodierbaren Feststoffmasse.

Die Vorrichtung soll es ermöglichen, in flüssigen Medien, beispielsweise Körperflüssigkeiten wie Magensäure Wirkstoffe mit vorgegebenem Geschwindigkeitsprofil freizusetzen, um dabei über einen längeren Zeitraum bestimmte Plasmaspiegel zu erzielen. Dabei können Wirkstoffe zum Beispiel nach peroraler Applikation mit kontrollierter Geschwindigkeit an den Magen- und Darmsaft abgegeben werden. Nach Applikation durch Implantation ist die interstitielle Gewebeflüssigkeit das Medium, mit welchem die Vorrichtung in unmittelbarem Kontakt stehend die Wirkstoffe abgibt. Eine weitere Verwendungsmöglichkeit ist die gezielte Freisetzung von Wirkstoffen, die dem Pflanzenschutz und/oder dem Pflanzenwuchs dienen, an Flüssigkeiten, die von Pflanzen aufgenommen werden. Das kann beispielsweise dadurch geschehen, daß erfindungagemäße Vorrichtungen in Behältnisse oder Leitungen eingebracht werden, in denen sich Spritz- oder Bewässerungsflüssigkeit befindet. Auch die direkte Applikation in den Erdboden ist im Sinne der Erfindung denkbar, weil dort in der Regel genügend Kapillarwasser vorhanden ist, um eine Erosion der erodierbaren Masse und damit die Freisetzung von Wirkstoff zu ermöglichen. Eine weitere denkbare Anwendungsmöglichkeit ergibt sich aus kontrollierter Freisetzung von antibakteriellen Wirkstoffen, insbesondere in Spül- oder Wascheinrichtungen für häuslichen oder klinischen Gebrauch.

Mit Vorrichtungen mit kontrollierter Wirkstofffreisetzung zum Beispiel bei pharmazeutischen Peroralia wird häufig das Ziel verfolgt, Wirkstoffe mit kurzer biologischer Halbwertzeit in einer Weise zu verabreichen, die bei geringer Einnahmefrequenz größere Schwankungen der Plasmakonzentrationen vermeidet. Dieses Ziel wird in der Regel durch Vorrichtungen erreicht, die Wirkstoffe über einen längeren Zeitraum mit gleichmäßiger Geschwindigkeit freisetzen. In einigen Fällen jedoch führen auch Veränderungen der Freisetzungsgeschwindigkeit zu relativ konstanten Plasmaspiegeln, beispielsweise wenn ein Wirkstoff in verschiedenen Abschnitten des Gastrointestinaltraktes mit sehr unterschiedlicher Geschwindigkeit resorbiert wird.

Die Vorteile geringer Konzentrationsschwankungen im Plasma sind sowohl die Vermeidung toxischer Effekte, die sich aus den hohen Maximalkonzentrationen nach Einnahme konventioneller Peroralia ergeben können, als auch eine Verlängerung der therapeutischen Wirkung. Ferner fördert eine verringerte Einnahmefrequenz die Zuverlässigkeit der Patienten hinsichtlich der regelmäßigen Einnahme.

Es ist bekannt, daß sich eine kontrollierte Wirkstofffreisetzung in manchen Fällen durch physikochemische Maßnahmen erzielen läßt, denen ein Wirkstoff unterworfen wird. Zu solchen Maßnahmen zählen der Einsatz von Adsorbaten, schwerlöslichen Salzen oder Komplexen. Eine größere Kontrolle über das Ausmaß der Retardierung wird in der Regel jedoch durch galenische Maßnahmen erzielt. Die Mehrzahl der bekannten Vorrichtungen zur kontrollierten Wirkstofffreisetzung lassen sich zwei Typen zuordnen, nämlich den Matrixsystemen und den Membransystemen. Matrixsysteme enthalten Wirkstoffe in gelöster oder dispergierter Form, seltener auch in Form eines multipartikulären pharmazeutischen Zwischenproduktes. Die Freisetzung geschieht entweder durch die Diffusion von Wirkstoffen aus der Matrix heraus oder durch die Erosion der Matrix. Membransysteme hingegen bestehen aus einem wirkstoffhaltigen Reservoir, welches mit einem für den Wirkstoff permeablen Polymerfilm umhüllt ist. In ihrem Fall geschieht die Freisetzung durch Diffusion durch die Membran.

Die Freisetzungsgeschwindigkeit hängt von verschiedenen Einflußgrößen ab. Bei Matrixsystemen gehören dazu u. a. spezifische Eigenschaften der verwendeten Stoffe wie Molmasse, Löslichkeit, Quellfähigkeit und Glasübergangstemperatur, aber auch die Wirkstoffkonzentration und die geometrische Form der Matrix. Bei der Freisetzung durch Diffusion zählen die Größe der Oberfläche, das Matrixvolumen, der Diffusionskoeffizient, die Konzentration und die Löslichkeit eines Wirkstoffes in der Matrix, die Porosität und Tortuosität der Matrix und der Diffusionswiderstand zwischen Matrix und einem flüssigen Umgebungsmedium zu den wesentlichen Faktoren. Membransysteme setzen Wirkstoffe mit einer Geschwindigkeit frei, die im wesentlichen von der Größe der Oberfläche, der Permeabilität des Wirkstoffes in der Membran und vom Konzentrationsgefälle zu beiden Seiten der Membran abhängt.

Bekannt sind schon Matrix- und Membransysteme, welche durch ihre spezielle geometrische Formgebung die Freisetzungsgeschwindigkeit beeinflussen. Hierbei handelt es sich um Vorrichtungen, bei denen eine im Freisetzungsverlauf wandernde Erosionsfront ihre Dimension verändert. Beispiele hierfür sind Vorrichtungen, deren Erosionsfläche zum Erhalt einer konstanten Freisetzungsgeschwindigkeit größer wird (Brooke, DE-0 24 48 631; McMullen, Eur.Pat.Appl. 0 259 219; Chopra et al., Eur.Pat.Appl. 0 542 364). Zur Erzielung eines gegenteiligen Effektes, nämlich der kontrollierten Herabsetzung der Freisetzungsgeschwindigkeit, wurde ein Reservoir beschrieben, dessen Erosionsfront im Freisetzungsverlauf kleiner wird (Herrmann, DE 38 09 978).

Ferner sind Vorrichtungen bekannt, welche eine Kontrolle der Freisetzungsgeschwindigkeit dadurch erzielen, daß sie eine oder mehrere wirkstofffreie und weitgehend undurchlässige Schichten enthalten,welche einen Teil ihrer Oberfläche bedecken (Zaffaroni, Eur.Pat.Appl. 0 127 282; Graham et al., US Pat. 4 814 182; Conte et al., Eur.Pat.Appl. 0 432 607).

WO 94/07470, das zum Stand der Technik gemäß Art. 54(3) und (4) EPÜ gehört, offenbart Vorrichtungen zur Kontrollierten Wirkstofffreisetzung, enthaltend eine Matrix und eine erodierbare Feststoffschicht mit einem sich von Null unterscheiden den Dickegradienten, wobei kugel-, ellipsen-, zylinder- und rechteckprismen förmige Matrices mit Kugel-, ellipsen-, zylinder- und rechtecksprismen förmigen Feststoffschichten Kombiniert sind. Derartige zusammensetzungen sind in den vorliegenden Ansprüchen ausgenommen.

Alternativ zu den weitverbreiteten Matrix- und Membransystemen wurden spezielle Vorrichtungen zur kontrollierten Freisetzung von Wirkstoffen entwickelt, welche ein Wirkstoffreservoir enthalten, in dem sich nach Zutritt von Wasser ein osmotischer Druck aufbaut. Die Membranen, die die Wirkstoffreservoirs umgeben, sind semipermeabel, das heißt, sie ermöglichen den Zutritt von Wasser, sind für Wirkstoffe jedoch undurchlässig, besitzen aber eine mikroskopische kleine Öffnung, durch welche eindiffundiertes Wasser wie auch gelöster Wirkstoff austreten können. Ein Vorteil solcher osmotisch aktiven Vorrichtungen ist, daß sie über längere Zeit sehr konstante Freisetzungegeschwindigkeiten erzielen können (Theeuwes, Pharm.Int. 5, S. 293, 1984).

Oft stößt die Verwirklichung von kontrollierten Freisetzungsgeschwindigkeiten mit herkömmlichen Matrix- oder Membransystemen auf Schwierigkeiten. Bei erodierbaren Matrices verlangsamt sich - je nach Form der Matrix - die Freisetzungsgeschwindigkeit im Verlaufe der Freisetzung mehr oder weniger stark aufgrund der Verkleinerung der erodierbaren Oberfläche. Die weiter oben bereits zitierten Vorrichtungen, die eine Verlangsamung durch die spezielle geometrische Formgebung der Matrix kompensieren, sind technisch schwer zu realisieren. Bei Diffusionsmatrices bildet sich dagegen im Freisetzungsverlauf eine durch zunehmende Wirkstoffdepletion kontinuierlich wachsende Diffusionsschicht mit der Folge aus, daß sich die Freisetzungsgeschwindigkeit in Abhängigkeit von t^{1/2} verringert (Higuchi, J. Pharm. Sci. 50, S. 874, 1961).

Membransysteme können theoretisch solange eine konstante Freisetzungsgeschwindigkeit aufweisen, wie das Reservoir den Wirkstoff in seiner Sättigungskonzentration gelöst enthält. Das ist dann der Fall, wenn zusätzlich zum gelösten auch ungelöster Wirkstoff in der Vorrichtung enthalten ist, welcher sich im Reservoir schnell genug auflöst, um bereits freigesetzten Wirkstoff zu ersetzen. Sobald die Sättigungskonzentration unterschritten wird, verlangsamt sich die Freisetzung proportional zur Abnahme des Konzentrationsgradienten zu beiden Seiten der Membran.

Die oben aufgeführten osmotisch gesteuerten Systeme setzen Wirkstoffe zwar mit konstanter Geschwindigkeit frei; ihre Herstellung erfordert jedoch den Einsatz einer besonders aufwendigen Technologie. Ein weiterer Nachteil solcher Vorrichtungen ist das Risiko gastrointestinaler Schleimhautschädigungen nach peroraler Anwendung. Schließlich sind sie weitgehend ungeeignet in allen Fällen, in denen ein anderes Freisetzungsprofil als jenes mit konstanter Geschwindigkeit gewünscht wird.

Der Erfindung liegt die Aufgabe zugrunde, eine Vorrichtung zu schaffen, die Wirkstoffe mit kontrollierter Geschwindigkeit in ein flüssiges Medium freisetzt, jedoch unter Umgehung der oben ausgeführten Nachteile des Standes der Technik.

Die Lösung gelingt bei einer Vorrichtung der im Oberbegriff von Anspruch 1 genannten Art mit der Erfindung entsprechend den im Kennzueichnungsteil genannten Merkmalen.

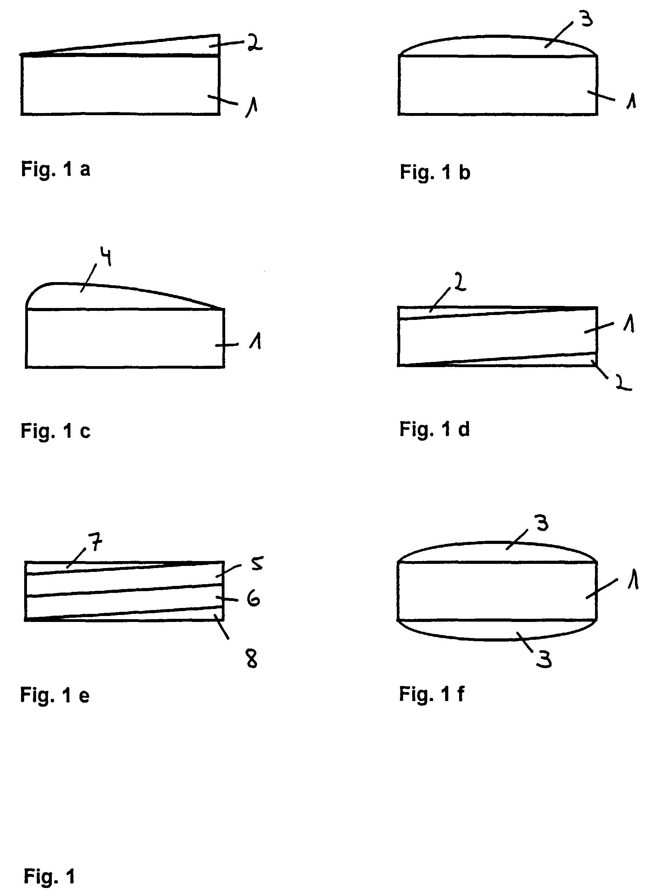

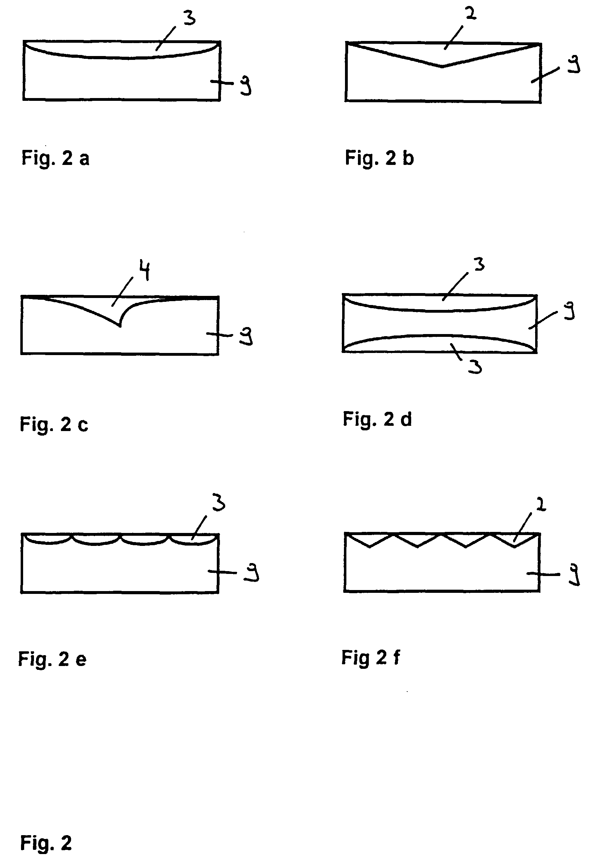

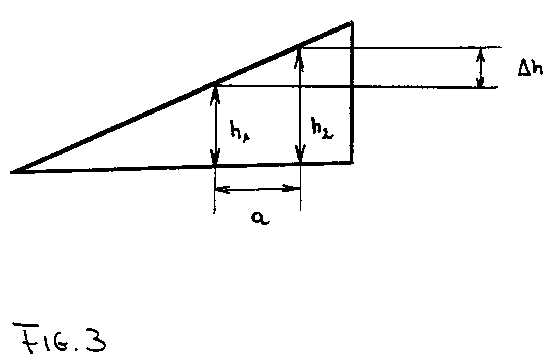

Unter einem "Dickegradienten" soll erfindungsgemäß eine geometrische Formgebung der Masse verstanden werden (Fig. 3 und 2), durch welche die erodierbare Masse an verschiedenen Stellen eine unterschiedliche Dicke h aufweist. Die Veränderung der Dicke über eine Längeneinheit a der erodierbaren Masse, also Δh/Δa, ist ein Dickegradient. Eine erfindungsgemäße erodierbare Masse kann einen einzigen Dickegradienten haben (wie in Fig. 1a); dort bleibt Δh/Δa konstant. Ändert sich Δh/Δa über die Strecke a kontinuierlich (wie in Fig. 1b), so gibt es für jede Stelle einen anderen Dickegradienten. Die Erfindung besteht in dem gezielten Einsatz von Dickegradienten, so daß eine erodierbare Masse in der beabsichtigten Geschwindigkeit erodiert wird und dadurch eine Vergrößerung der Kontaktfläche der wirkstoffhaltigen Matrix zur Umgebungsflüssigkeit bewirkt.

Der Begriff "Erosion" hat sich in der Pharmazeutischen Technologie für alle Prozesse durchgesetzt, bei denen Feststoffmassen "abgetragen" werden. Dabei ist es nicht entscheidend, ob die Massenreduktion eines festes Körpers dadurch zustande kommt, daß feste Bestandteile in Lösung gehen und infolgedessen abdiffundieren, oder ob zunächst ein chemischer Abbau stattfindet, bei dem z.B. lange Polymerketten zu besser löslichen Oligomeren, Monomeren oder sonstigen Abbauprodukten gespalten werden.

Bei einer erfindungsgemäßen Vorrichtung steht zu Beginn der Wirkstofffreisetzung höchstens ein Teil der Matrixoberfläche in Kontakt mit einem flüssigen Außenmedium und damit als Freisetzungafläche zur Verfügung. Im weiteren Freisetzungsverlauf verringert sich die Wirkstoffkonzentration in der Matrix. Dieser Effekt führt jedoch nicht wie bei herkömmlichen Matrixsystemen zur Herabsetzung der Freisetzungsgeschwindigkeit, da gleichzeitig die Erosion der mit der Matrix in Kontakt stehenden erodierbaren Schicht voranachreitet, wodurch sich die Freisetzungsfläche der Matrix kontinuierlich vergrößert. Der Zunahme der Freisetzungsfläche über die Zeit kommt somit eine entscheidende Bedeutung hinsichtlich der Kontrolle der Freisetzungsgeschwindigkeit zu. Beispielsweise erhält man eine Freisetzung nach nullter Ordnung, wenn die Zunahme der Freisetzungsfläche den Effekt der abnehmenden Wirkstoffkonzentration genau kompensiert.

Besonders vorteilhaft ist die erfindungsgemäße Lösung der Aufgabe hinsichtlich ihrer Einfachheit und Flexibilität: Erodierbare Massen mit Dickegradienten, welche die Erosionsgeschwindigkeit steuern, lassen sich mit allen gängigen pharmazeutisch-technologischen Produktionstechniken erzeugen. Wirkstoffhaltige Matrices verschiedener Formulierungstypen lassen sich durch Aufbringen einer erfindungsgemäßen erodierbaren Masse zu Vorrichtungen mit kontrollierter Wirkstofffreisetzung gestalten. Die häufig gewünschte Freisetzung mit konstanter Geschwindigkeit - wie auch andere Freisetzungsprofile - ist sehr exakt zu realisieren. Abweichungen vom gewünschten Freigabeprofil lassen sich auch nach der Festlegung auf eine bestimmte Rezeptur durch Modifizierung der Form der erodierbaren Masse korrigieren.

Die erodierbare Masse besteht bei Verwendung der Vorrichtung als Arzneimittel in der Regel aus physiologisch unbedenklichen Polymeren oder wachsartigen Substanzen und ggf. weiteren pharmazeutischen Hilfsstoffen. Beispiele für solche Polymere sind Polysaccharide wie Gummen, Stärke- oder Cellulosederivate, Polyacrylate und -methacrylate, Polylaktide, Polyglykolide, Polyoxyethylene und Polyoxypropylene, Proteine, Polyvinylalkohol, Polyvinylacetat, Polyvinylchlorid oder Polyvinylpyrrolidon. Wachsartige Substanzen sind z.B. hydriertes Ricinusöl oder Cetylstearylalkohol. Weitere pharmazeutische Hilfsstoffe können aus den Gruppen der Stabilisatoren, Lösungsvermittler, Tenside, Füllstoffe, Weichmacher, Hydrophilisierungsmittel, Pigmente bzw. Farbstoffe, Substanzen zur Einstellung des pH-Wertes, Fließregulierungsmittel, Formtrennmittel, Schmiermittel usw. stammen. Je nach Kompatibilität und gewünschter Erosionsgeschwindigkeit muß der Anteil der einzelnen Komponenten eingestellt werden.

Bei Verwendung im Pflanzenschutz o.ä. spielt die physiologische Unbedenklichkeit im Gegensatz zur biologischen Abbaubarkeit keine wesentliche Rolle.

Eine einfache Möglichkeit, eine erfindungsgemäße Vorrichtung herzustellen, besteht darin, daß die wirkstoffhaltige Matrix selbst schichtförmig hergestellt und danach mit mindestens einer erodierbaren Schicht zumindest teilweise beschichtet wird.

Fig. 1 zeigt erfindungsgemäße Vorrichtungen mit schichtförmigem Aufbau der wirkstoffhaltigen Matrix 1. Die wirkstoffhaltige Matrix kann wie in Fig. 1a bis 1c eine oder wie in Fig. 1d bis 1e mehrere erodierbare Schichten aufweisen. In einigen Fällen wird es zur Steuerung der Freisetzungsgeschwindigkeit ausreichen, die erodierbare Schicht oder Schichten wie in Fig. 1a, 1d und 1e keilförmig 2 zu konstruieren; in anderen Fällen werden kugelsegmentartige erodierbare Schichten 3 wie in Fig. 1b und 1f vorzuziehen sein, um die Freisetzungsgeschwindigkeit sehr exakt zu steuern und jedem gewünschten Verlauf und jedem Matrixtyp anpassen zu können, besteht auch die Möglichkeit, wie in Fig. 1c gezeigt mindestens eine erodierbare Schicht mit Dickegradienten, welche sich über deren Erstreckung nach komplexen Funktionen ändert 4, einzusetzen.

In manchen Fällen kann es zweckmäßig erscheinen, daß die wirkstoffhaltige Matrix mehrschichtig ist. Das ist z.B. dann der Fall, wenn die Vorrichtung mehr als einen Wirkstoff enthält und unterschiedlich geformte erodierbare Schichten zur Erzielung des gewünschten Freigabeprofils notwendig sind. Fig. 1e zeigt eine Vorrichtung mit einer zweischichtigen wirkstoffhaltigen Matrix bestehend aus den beiden Untereinheiten 5 und 6, mit einer erodierbaren Schicht 7, welche die Wirkstofffreisetzung aus der einen Matrixuntereinheit 5 steuert, und eine andere erodierbare Schicht 8, welche das Freisetzungsprofil aus der zweiten Matrixuntereinheit 6 kontrolliert.

Verfahrenstechnisch kann es günstig sein, die erodierbare Schicht bzw. Schichten auf wirkstoffhaltige Matrices aufzubringen, welche in ihrer Form modifiziert wurden.

Fig. 2 zeigt erfindungsgemäße Vorrichtungen, die Matrices mit unebener Oberfläche 9 enthalten, wobei die sich daraus ergebenden Vertiefungen in der Matrixoberfläche von den erodierbaren Schichten ausgefüllt werden. Fig. 2a zeigt eine erfindungsgemäße Vorrichtung mit unebener Matrix 9 und einer kugelsegmentartigen erodierbaren Schicht 3, Fig. 2d und 2e ebensolche mit mehreren derartigen Schichten. Analog zeigen Fig. 2b und 2f Vorrichtungen mit unebener Matrix 9 und einer oder mehreren keilförmigen erodierbaren Schichten 2. Fig. 2c zeigt die Kombination einer unebenen Matrix mit einer erodierbaren Schicht mit Dickegradienten, welche sich über die Erstreckung der Schicht nach komplexen Funktionen ändern 4. In allen in Fig. 2 gezeigten Beispielen passen sich die erodierbaren Schichten den Konturen der Matrixoberfläche an. Dadurch kann die äußere Form der Vorrichtungen ungeachtet der Form der erodierbaren Schicht und der Matrix regelmäßig sein.

Wirkstoffhaltige Matrices, die sich als Bestandteil der erfindungsgemäßen Vorrichtungen eignen, lassen sich mit vielen gängigen pharmazeutischen Herstellungstechnologien fertigen. So lassen sich durch Komprimieren von Pulvern, Pulvermischungen oder Granulaten mit gängigen Tablettierwerkzeugen schichtförmige Matrices produzieren. Auch mehrschichtige Matrices lassen sich durch Komprimieren fertigen. Dünne Matrices lassen sich beispielsweise über Gieß- oder Beschichtungsverfahren herstellen, bei denen Lösungen oder Suspensionen in dünner Schicht meist auf ein intermediäres Trägermaterial aufgebracht und getrocknet werden. Weiterhin sind zur Herstellung der Matrices solche Verfahren geeignet, bei denen Schmelzen eingesetzt werden. Dazu gehören z.B. Spritzguß- und Extrusionsverfahren.

Die erodierbaren Schichten können erfindungsgemäß ebenfalls mit allen Verfahren gefertigt werden, die auch zur Herstellung der wirkstoffhaltigen Matrices zur Verfügung stehen. Dabei ist es unerheblich, ob wirkstoffhaltige Matrix und erodierbare Schicht nacheinander oder simultan erzeugt werden. In Fällen, in denen die Haftung zwischen Matrix und erodierbarer Schicht ungenügend ist, kann es erforderlich sein, haftungsverbessernde Zusätze zu verwenden. Bei diesen Zusätzen handelt es sich um physiologisch unbedenkliche Polymere mit adhäsiven Eigenschaften, welche auch in Gegenwart von Wasser erhalten bleiben.

Erfindungsgemäße Vorrichtungen lassen sich unter anderem dazu verwenden, pharmazeutische Wirkstoffe zu therapeutischen oder diagnostischen Zwecken zu verabreichen und dabei gleichmäßige Plasmaspiegel zu erzielen. Eine weitere Verwendungsmöglichkeit ist die gezielte Freisetzung von Wirkstoffen, die dem Pflanzenschutz oder dem Pflanzenwuchs dienen, an Flüssigkeiten, die von Pflanzen aufgenommen werden oder zur Abgabe antibakterieller Stoffe für Wasch- und/oder Spülanlagen (WC) im häuslichen, industriellen (Flaschenwaschanlagen) oder klinischen Bereichen.

## Patentansprüche

1. Vorrichtung zur kontrollierten Freisetzung von Wirkstoffen in flüssigen Medien aus einer wirkstoffhaltigen, Wirkstofffreisetzungsflächen aufweisenden Matrix,die den im Matrixmaterial enthaltenen Wirkstoff durch dessen Permeation und Freigabe über die flüssigkeitszugängliche Matrixoberfläche in retardierter gesteuerter Form abgibt, dadurch gekennzeichnet, daß die Wirkstofffreisetzungsflächen der Matrix mindestens teilweise von einer in flüssigen Medien erodierbaren Feststoffschicht abgedeckt sind, die ein solches Dickeprofil mit mindestens einem sich von Null unterscheidenden Dickegradienten aufweist, daß während der Erosion dieser Feststoffschicht im flüssigen Medium eine die Wirkstofffreisetzungsrate mitbestimmende Freilegung zunächst abgedeckter Teile der Matrix erfolgt, wobei eine Kombination von Kugel-, Ellipsen-, Zylinder- oder Rechteckprismenform der Matrix mit Kugel-, Ellipsen-, Zylinder- oder Rechtwinkelprismenform der erodierbaren Feststoffschicht ausgeschlossen ist.

2. Vorrichtung zur kontrollierten Freisetzung von Wirkstoffen nach Anspruch 1, dadurch gekennzeichnet, daß die wirkstoffhaltige Matrix mit einer unebenen Fläche mit der erodierbaren Masse in Kontakt steht.

3. Vorrichtung zur kontrollierten Freisetzung von Wirkstoffen nach Anspruch 1, dadurch gekennzeichnet, daß die Kontaktfläche der erodierbaren Masse den Konturen der Matrix angepaßt ist.

4. Vorrichtung zur kontrollierten Freisetzung von Wirkstoffen nach einem der Ansprüche 1 bis 3, dadurch gekennzeichnet, daß die Matrix aus einer oder mehreren Schichten besteht.

5. Vorrichtung zur kontrollierten Freisetzung von Wirkstoffen nach einem der Ansprüche 1 bis 4, dadurch gekennzeichnet, daß die Haftung zwischen Matrix und erodierbarer Masse durch Zusätze erzeugt ist, wobei es sich bei den Zusätzen um physiologisch unbedenkliche Polymere mit adhäsiyen Eigenschaften handelt, die auch in Gegenwart von Flüssigkeiten erhalten bleiben.

6. Vorrichtung zur kontrollierten Freisetzung von Wirkstoffen nach einem der Ansprüchen 1 bis 5, dadurch gekennzeichnet, daß die Matrix mindestens eine erodierbare Masse mit unterschiedlichen Dickegradienten aufweist.

7. Vorrichtung zur kontrollierten Freisetzung von Wirkstoffen nach Ansprüchen 1 bis 6, gekennzeichnet durch eine Teilabdeckung der Matrix durch mindestens eine keilförmige erodierbare Masse.

8. Vorrichtung zur kontrollierten Freisetzung von Wirkstoffen nach Ansprüchen 1 bis 6, gekennzeichnet durch eine TEilabdeckung der Matrix durch mindestens eine kugelsegmentartige erodierbare Masse.

9. Vorrichtung zur kontrollierten Freisetzung von Wirkstoffen nach einem oder mehreren der Ansprüche 1 bis 8, dadurch gekennzeichnet, daß die erodierbare Masse Wirkstoff enthält.

10. Verwendung der Vorrichtung nach einem oder mehrerender vorangehenden Ansprüche zur Herstellung von Arzneimittel.

11. Verwendung der Vorrichtung nach einem oder mehreren der vorangehenden Ansprüche zur kontrollierten Freisetzung von Pflanzenschutzmitteln.

12. Verwendung der Vorrichtung nach einem oder mehreren der vorangehenden Ansprüche zur kontrollierten Freisetzung von Düngestoffen.

13. Verwendung der Vorrichtung nach einem oder mehreren der vorangehenden Ansprüche zur kontrollierten Freisetzung von antibakteriellen Stoffen in Bereichen außerhalb der chirurgischen und therapeutischen Behandlung von Mensch und Tier und von am menschlichen und tierischen Körper vorgenommenen Diagnostizierrerfahren, insbesondere in Spül- oder Wascheinrichtungen.

## Claims

1. A device for the controlled release of active substances in liquid media from an active substance-containing matrix having active substance-releasing areas, which matrix releases the active substance contained in the matrix material in a retarded, controlled manner by way of the permeation and release of the active substance via the liquid-accessible matrix surface, characterized in that the active substance-releasing surfaces of the matrix are covered at least partially by a solid matter layer erodible in liquid media which has a thickness profile with at least one thickness gradient different from zero such that during the erosion of the said solid matter layer in the liquid medium an exposure of initially covered matrix parts takes place, which exposure contributes to the control of the active substance release rate, with a combination of a spheroid, ellipsoid, cylindrical or rectangular prismatic shape of the matrix with a spheroidal, ellipsoidal, cylindrical or rectangular prismatic shape of the erodible solid matter layer being excluded.

2. The device for the controlled release of active substances according to claim 1, characterized in that the active substance-containing matrix has an uneven surface which is in contact with the erodible mass.

3. The device for the controlled release of active substances according to claim 1, characterized in that the contact surface of the erodible mass is adapted to the contours of the matrix.

4. The device for the controlled release of active substances according to any one of claims 1 to 3, characterized in that the matrix consists of one or several layers.

5. The device for the controlled release of active substances according to any one of claims 1 to 4, characterized in that the adhesion between matrix and erodible mass is created by additives which are physiologically acceptable polymers having adhesive properties continuing to exist even in the presence of liquids.

6. The device for the controlled release of active substances according to any one of claims 1 to 5, characterized in that the matrix has at least one erodible mass with different thickness gradients.

7. The device for the controlled release of active substances according to claims 1 to 6, characterized by a partial covering of the matrix by at least one wedge-shaped erodible mass.

8. The device for the controlled release of active substances according to claims 1 to 6, characterized by a partial covering of the matrix by at least one erodible mass having the shape of a spherical segment.

9. The device for the controlled release of active substances according to one or several of claims 1 to 8, characterized in that the erodible mass contains active substance.

10. The use of the device according to one or several of the preceding claims for the production of drugs.

11. The use of the device according to one or several of the preceding claims for the controlled release of plant protectants.

12. The use of the device according to one or several of the preceding claims for the controlled release of fertilizers.

13. The use of the device according to one or several of the preceding claims for the controlled release of antibacterial substances in areas outside the surgical and therapeutic treatment of humans and animals and the diagnostic methods employed on the human and animal body, in particular in flushing or washing installations.

## Revendications

1. Dispositif de libération contrôlée de principes actifs dans des milieux liquides, hors d'une matrice contenant les principes actifs et présentant des surfaces de libération de principes actifs, laquelle matrice libère de manière contrôlée et retardée le principe actif contenu dans le matériau de matrice par perméation et libération à travers la surface de la matrice accessible au liquide, caractérisé en ce que les surfaces de libération du principe actif de la matrice sont recouvertes au moins en partie par une couche solide érodable dans le milieu liquide, qui présente un profil d'épaisseur ayant au moins un gradient d'épaisseur différent de zéro, en ce que pendant l'érosion de cette couche solide dans le milieu liquide, une libération déterminant le taux de libération du principe actif s'effectue d'abord par les parties découvertes de la matrice, une combinaison d'une forme sphérique, elliptique, cylindrique ou en prisme rectangulaire de la matrice avec une couche solide érodable de formes sphérique, elliptique, cylindrique ou en forme de prisme rectangulaire étant exclue.

2. Dispositif de libération contrôlée de principes actifs selon la revendication 1, caractérisé en ce que la matrice contenant les principes actifs est en contact avec la masse érodable par l'intermédiaire d'une surface irrégulière.

3. Dispositif de libération contrôlée de principes actifs selon la revendication 1, caractérisé en ce que la surface de contact de la masse érodable est adaptée aux contours de la matrice.

4. Dispositif de libération contrôlée de principes actifs selon l'une des revendications 1 à 3, caractérisé en ce que la matrice est constituée d'une ou de plusieurs couches.

5. Dispositif de libération contrôlée de principes actifs selon l'une des revendications 1 à 4, caractérisé en ce que l'adhérence entre la matrice et la masse érodable est créée par des additifs, les additifs étant des polymères physiologiquement acceptables présentant des propriétés adhésives qui se maintiennent même en présence de liquides.

6. Dispositif de libération contrôlée de principes actifs selon l'une des revendications 1 à 5, caractérisé en ce que la matrice présente au moins une masse érodable présentant différents gradients d'épaisseur.

7. Dispositif de libération contrôlée de principes actifs selon les revendications 1 à 6, caractérisé par un recouvrement d'une partie de la matrice par au moins une masse érodable en forme de biseau.

8. Dispositif de libération contrôlée de principes actifs selon les revendications 1 à 6, caractérisé par un recouvrement d'une partie de la matrice par au moins une masse érodable en forme de calotte sphérique.

9. Dispositif de libération contrôlée de principes actifs selon l'une ou plusieurs des revendications 1 à 8, caractérisé en ce que la masse érodable contient un principe actif.

10. Utilisation du dispositif selon l'une ou plusieurs des revendications précédentes pour la préparation de médicaments.

11. Utilisation du dispositif selon l'une ou plusieurs des revendications précédentes pour la libération contrôlée d'agents de protection des plantes.

12. Utilisation du dispositif selon l'une ou plusieurs des revendications précédentes pour la libération contrôlée d'agents fertilisants.

13. Utilisation du dispositif selon l'une ou plusieurs des revendications précédentes pour la libération contrôlée de produits anti-bactériens dans les domaines extérieurs au traitement chirurgical et thérapeutique de l'homme et de l'animal et aux procédés de diagnostic exécutés sur le corps humain et animal, en particulier dans des installations de rinçage ou de lavage.
